# EUROPEAN PATENT APPLICATION

(11) **EP 3 858 354 A1**
(43) Date of publication of application: **04.08.2021**
(21) Application number: 19866151.4
(22) Date of filing: 20.09.2019
(51) Int. Cl.: A61K 31/47, A61K 31/38, A61P 11/06, A61P 37/08

(54) **SYNERGIC PHARMACEUTICAL COMBINATION OF A LEUKOTRIENE-RECEPTOR ANTAGONIST AND AN INVERSE AGONIST OF HISTAMINE H1**

(30) Priority: 26.09.2018 MX 2018011699
(71) Applicant: Amézcua Amézcua, Federico, Guadalajara, Jalisco, 44898 (MX); Amézcua Amézcua, Carlos, Guadalajara, Jalisco, 44898 (MX)
(72) Inventor: GARCÍA ARMENTA, Patricia del Carmen, Zapopan, Jalisco, 45019 (MX); CHÁVEZ GARCÍA, José Alonso, Zapopan, Jalisco, 45138 (MX)
(74) Representative: Ungria López, Javier
(86) International application number: PCT/MX2019/000100
(87) International publication number: WO 2020/067866

(57) **Abstract**

The present invention relates to a pharmaceutical composition comprising the synergic combination of a leukotriene-receptor antagonist, such as the active substance montelukast and its pharmaceutically acceptable salts, and an inverse antagonist of histamine H1, such as the active substance ketotifen and its pharmaceutically acceptable salts, which are formulated with pharmaceutically acceptable additives and/or excipients and/or vehicles in a single dosing unit to be administered orally, transdermally or by devices for oral or nasal inhalation. The composition is suitable for the treatment of atopic diseases such as asthma, allergic rhinitis and atopic dermatitis.

## Description

### FIELD OF THE INVENTION

The present invention is related to the technical field of the pharmaceutical industry, and its object is to provide a pharmaceutical composition comprising the synergic pharmaceutical combination of a leukotriene-receptor antagonist, constituted by the active ingredient montelukast or pharmaceutically acceptable salts thereof and an inverse antagonist of histamine H1, constituted by the active ingredient ketotifen or a pharmaceutically acceptable salt thereof, which are administered with pharmaceutically acceptable excipients, adjuvants or additives, formulated in a single solid or liquid dosing unit to be administered orally, for pharmacological uses in the treatment of an atopic disease such as asthma, allergic rhinitis and atopic dermatitis.

The combination of the aforementioned active principles exhibits improved properties, and provides a greater therapeutic effect if said active principles are jointly administered as a single dosing unit instead of being administered separately, providing benefits such as a lower administered dose, greater therapeutic effect and less adverse effects.

### BACKGROUND OF THE INVENTION

All manifestations of allergic pathology, such as asthma, allergic rhinitis and atopic dermatitis share the same immunologic mechanism of atopic diseases. The release of chemical mediators such as histamines and leukotrienes, among others, and of cytokines, originated by the activation of mast cells sensitized by a specific IgE allergen, are responsible of symptoms in patients when interacting with vascular and nervous receptors.
According to international epidemiologic studies, asthma is a chronic respiratory disease most prevalent during childhood and adolescence and is defined by the Global Initiative for Asthma (GINA) as a chronic airway inflammation, in which certain cells and cellular mediators perform an important role. This chronic inflammation is associated with an increase in airway hyperreactivity, resulting in recurrent episodes of wheezing, dyspnea, chest discomfort and coughing, more frequently during night or early morning. Asthma is considered a syndrome comprising several disease forms, and its clinical manifestations are caused by the interaction of genetic and environmental factors. Its clinical manifestations can be very variable, with symptoms that can be either acute and sporadic or chronic, seasonal or exercise-related, or even a serious persistent disease. According to reports from the World Health Organization, asthma is a disease affecting at least 235 million people globally, and it is predicted that in the next 10 years deaths could increase due to this illness, its consequences and treatment malpractices.

Asthma is a clear example of a complex disease, since it is characterized by a multi-faceted etiology and intertwined subjacent mechanisms. Any disease termed as "complex" is associated to a general notion that it lacks an adequate understanding, and asthma is no exception to this notion. In fact, it is more appropriate to refer to this entity as a syndrome and not as an illness, since it is defined more by its clinical characteristics than by its subjacent mechanisms, and even which of these mechanisms are the most important is still subject to an intense debate. Asthma is characterized by an abnormal accumulation of immune cells and airway activation, and by the dysfunction of specialized parenchymal cells. The main features related to breathing are reversible respiratory flow obstructions, lung hyperreactivity to smooth muscle agonists and airway inflammation. As was mentioned above, its etiology is multi-faceted and the disease can be triggered by factors as diverse as exercising, cold air, emotional stress and as a response to allergies, the latter leading the list of triggering mechanisms. The treatment for this illness or syndrome has focused on airway inflammation, which involves T-cells, mastocytes and eosinophils. While acute inflammation is a benign non-specific response of tissues to the harm that most often results in the reparation and restoration of normal structures and functions, asthma still represents an airway chronic inflammatory process followed by morphological changes known as obstructive remodeling.

The inflammatory response of asthma can be divided in several response phases, the first being the early-phase reaction. The stimulus caused by an inhaled allergen results in an early allergic inflammatory response, and in some instances, this can be followed by a late-phase reaction.

The onset of the early-phase reaction is when the allergen specific IgE carrying cells are activated. This is characterized by a rapid activation of airway mast cells and macrophages. Activated cells rapidly release proinflammatory mediators such as histamines, eicosanoids and reactive oxygen species that induce airways muscle contraction, mucus secretions and vasodilation.

Bronchial microcirculation has a central role in this inflammatory process. Inflammatory mediators induce microvascular liquid leakage with plasma exudation towards the airway. The acute plasmatic proteins leakage induces an enlarged, flooded and edematous airway walls, and, as a result, a narrowing of the airway inner diameter.

Moreover, plasma can also cross the epithelium, passing through tight cell junctions and accumulating in the airway inner diameter. The plasma exudate can compromise the epithelial integrity, and its presence in the airway inner diameter can reduce the depuration of mucus. Plasmatic proteins can also promote the formation of viscous exudate plugs, mixed with mucus, epithelial and inflammatory cells. Altogether, these events contribute to airway obstruction.

Late-phase reaction. This inflammatory phase occurs 6 to 9 hours after an allergen triggers the response, and involves the recruitment and activation of eosinophils, T helper cells, basophils, neutrophils and macrophages. There is a selective T cell retention of the airway, the expression of adhesion molecules and the release of proinflammatory mediators and cytokines involved in the recruitment and activation of more inflammatory cells. T cell activation after the allergen stimulus results in the release of TH2-cells, which could be the late response phase key mechanism.

Inflammatory cell recruitment inside the airway. The late response phase is considered a model system for studying the chronic inflammation mechanism of asthma.

The term "slow reacting substance of anaphylaxis" (SRS-A), coined by Brocklehurst in 1960, is still employed by many treating physicians. However, the chemical structure of SRS-A was not identified until 1979 by Samuelsson et al, who discovered it consisted in a family of biologically active fatty acids derived from the arachidonic acid metabolism in many cells, including eosinophils, mast cells and lymphocytes. The term "leukotriene" is adequate since it is synthesized in leukocytes and its chemical structure contains three conjugated double bonds, and thus is a triene. Leukotrienes C4, D4, and E4 are particularly important for asthma patients, and since they contain the amino acid cysteine as a residue, are usually referred to as cysteinyl leukotrienes.

Cysteinyl leukotrienes are very important biological mediators in asthma, interacting with at least one specific receptor in the lungs which already has been completely identified. The biological effects of the leukotriene's receptor activation comprise bronchoconstriction, hyperreactivity of the bronchial smooth muscle, inflammatory cells recruitment including eosinophils, increase of vascular permeability and mucus formation. Induced bronchoconstriction is very powerful and thus leukotrienes can induce several key effects in the physiopathology of asthma. Also, many studies have shown that leukotrienes are excessively produced in patients with asthma, reinforcing the view that they indeed are important biological mediators. Leukotrienes exert their biological effects through the union and activation of specific receptors located in the membranes of target cells. Presently two types of cysteinyl leukotrienes, cysLT1 and cysLT2, have been pharmacologically described and characterized, and are widely distributed in several kinds of cells, including bronchial smooth muscles cells and inflammatory cells such as eosinophils and mastocytes. The antagonists of these receptors haven been widely employed to treat asthma, since they have a unique mechanism of action as a result of anti-inflammatory and bronchodilation effects. The receptor antagonists exhibit a special affinity for the cysLT1 receptor, and they include montelukast, which is capable of markedly inhibiting exercise-induced bronchoconstriction, and the early- and late-phase reaction induced by allergen inhalation. Several clinical trials haven proved the efficacy of montelukast for alleviating persistent symptoms, even in those patients that still exhibit symptoms despite having been treated with high doses of inhaled corticosteroids.

Montelukast's relative efficacy versus other therapeutic alternatives, whether as substitutes of montelukast or combined with it, is still unclear and needs further and deeper research. Some reports have shown that such alternatives can significantly improve lung function and the control of symptoms, and that they are well tolerated with mild secondary effects.

Another mediator described in asthma is histamine. Histamine [2-(4-imidazol) ethylamine] was the first mediator involved in the physiopathological changes of the disease, when it was found that its administration induced many characteristics of the disease. Histamine is synthetized and released by mast cells in the airway walls, and by circulating and infiltrating basophils. Histamine causes several effects in the airway function mediated by specific receptors in the target cells' membranes. Three types of histamine receptors have been pharmacologically recognized; H1 and H2 receptors have 7 transmembrane domains and are coupled to G proteins. Histamine stimulates phosphoinositol hydrolysis in bronchial smooth muscles, and phosphoinositol hydrolysis is intimately related to receptor occupancy and the contractile response. Histamine also increases inositol-1,4,5-trisphosphate (IP3) concentrations in bronchial smooth muscles, which in turn increases calcium intracellular concentrations. Bronchoconstriction was one of the first identified effects of histamine. When administered by inhaled or i.v. route it causes bronchoconstriction, which is inhibited by H1 receptor antagonists (such as clorpheniramine, terfenadine or ketotifen). Histamine induces in vitro the contraction of both central and peripheral airways, with a more powerful effect in peripheral airways. Asthmatic patients are more sensitive than healthy subjects to the bronchoconstriction effects of inhaled or intravenous histamine, and this is a manifestation of hyperreactivity. There is a certain degree of baseline tone in human bronchial smooth muscle in vitro preparations which is decreased by H1 receptor antagonists, suggesting that histamine's baseline release (presumably derived from mast cells) contributes to this tone. This is consistent with the bronchodilation effects reported for intravenous or oral administration of H1 receptor antagonists in patients with asthma, although not in healthy subjects. Histamine causes a vasodilation response mediated by H1 receptors, and combined with an increase in blood flow, histamine causes plasma extravasation in postcapillary venules of bronchial circulation, and these effects are blocked by H1 receptors antagonists. Histamine also induces plasma exudation in the airways, although this exudation participates little in the airway narrowing induced by histamine. On the other hand, histamine stimulates mucus secretion in human airways in vitro preparations, but is not blocked by H1 antagonists. Histamine induces an increase of secretory IgA and lactoferrin, implying that it activates glandular secretions and that this effect is blocked by H1 antagonists.

Histamine also exerts effects over inflammatory cells, and it has been found that it impacts the release of cytokines and inflammatory mediators in a range of immune and inflammatory cells. Histamine is a selective chemoattractor for eosinophils, and also activates human eosinophils. Histamine stimulates beta-glucuronidase release by alveolar macrophages, and this effect is mediated by H1 receptors.

Histamine mediates most of its effects over the airway function, suggesting that H1 antagonists (such a ketotifen) could have a therapeutic effect for asthma. However, and even though they can be administered in large doses, a meta-analysis of several clinical trials could not establish their effectiveness in the treatment of patients with asthma. Antihistamines do cause a small bronchodilation in asthmatic patients, indicating a certain degree of histamine tone.

Histamine is produced by mast cells in asthmatic airways and causes many of the effects relevant for the physiopathological mechanisms of asthma, including bronchoconstriction, plasma exudation and mucus secretion, and there are evidences of effects on the inflammatory process. New alternatives in the use of antihistamines could potentially have a greater beneficial effect for asthma, and thus further research is needed.

Presently many treatments for asthma are available, including pharmacological treatments. The montelukast-ketotifen combination is an association that has potential therapeutic usefulness for asthmatic patients.

Presently many leukotriene-receptor antagonist drugs are also used, such as zafirlukast and pranlukast besides montelukast, while inverse H1 histamine agonists such as cetirizine, loratadine and ketotifen have a proven efficacy in the treatment of several asthma conditions and have caused less adverse effects.

The mechanism of action of montelukast is as a leukotriene D4 (LTD4) selective antagonist in the cysteinyl leukotriene receptor CysLT1 in the human airway. It inhibits the actions of LTD4 in the CysLT1 receptor, preventing airway edema, smooth muscle contraction and increased secretion of thick and viscous mucus.

Montelukast is the compound:
1-[[[(1R)-1-[3-[(1E)-2-(7-chloro-2-quinolinyl)ethenyl]phenyl]-3-[2-(1-hydroxy-1-methylethyl)phenyl]propyl]thio]methyl] cyclopropaneacetic acid, represented by Formula (I) 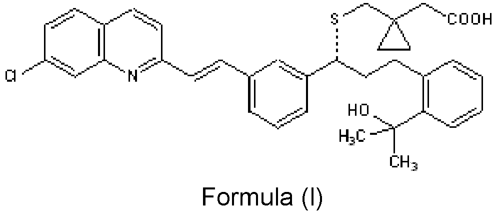 Disclosed for the first time in patent US 5,565,473 as an anti-asthmatic, antiallergic, anti-inflammatory and cytoprotective agent, it is also useful in the treatment of angina, cerebral vasospasm, glomerulonephritis, hepatitis, endotoxemia, uveitis and allogeneic transplant rejection.

Montelukast is administered orally as tablets, chewable lozenges or granules. It reaches maximum plasma concentration 2-4 hours after its oral administration, with a bioavailability of 64%. Its absorption is not modified by the ingestion of food. Plasma protein binding is 99%. It is extensively metabolized in the liver by the CYP3A4, CYP2A6 and CYP2C9 isoenzymes of cytochrome P450. The biological half-life of montelukast is 2.7 - 5.5 hours and is eliminated together with its metabolites almost exclusively in feces through the bile duct. Its metabolism decreases and its biological half-life increases if there is a mild hepatic dysfunction. Its pharmacokinetics is similar both in young and elderly patients and is not affected by kidney failure.

Ketotifen is a compound with chemical name 4,9-Dihydro-4-(1-methylpiperidin-4-ylidene)-10H-benzo(4,5)cyclohepta[1,2-b]thiophen-10-one, represented by Formula (II),

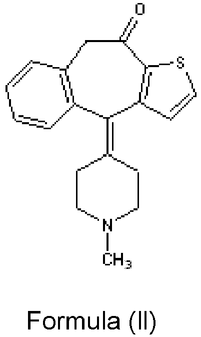

It was described for the first time in patent US 3,682,930 to be used as a selective antihistamine.

Ketotifen is an active oral benzo-cyclohepta-thiophene derivative that has been shown as capable of inhibiting passive cutaneous anaphylaxis by antagonizing slow reacting substances of anaphylaxis (SRS-A). It also exerts a stabilizing effect on mast cells, has little anticholinergic activity and increases intracellular cyclic APM (cAMP) by inhibiting phosphodiesterase. Its stabilizing effect on mast cells is similar to that of sodium cromoglycate; there is no cross-tolerance between these two drugs. Ketotifen is almost fully absorbed following oral administration. Its bioavailability is approximately 50% due to a first pass effect of the liver metabolism (glucuronidation and demethylation) of about 50%.

It reaches its maximum plasma concentration after 2 to 4 hours. Its therapeutic plasma concentrations are approximately 1-4 µg/ml. Its protein binding is 75% and is eliminated in a biphasic form, with a short half-life of 3-5 hours and a long half-life of 21 hours.

Approximately 1% is excreted unaltered in urine during the first 48 hours, and 60 to 70% as its metabolites, mainly the practically inactive N-glucuronide and the active form norketotifen.

Fecal excretion can be up to 30 to 40%. Although the metabolic pathway in children and adults is the same, the N-demethyl metabolite is higher in children, indicating that the ketotifen metabolism in children is significantly faster than in adults; therefore, the dose required by adults and children over 3 years old is the same. The bioavailability of ketotifen is not altered by food intake.

Ketotifen is an effective non-competitive blocker of histamine H1 receptors, which explains its efficacious antiallergic action. The mechanism of action of ketotifen for preventing bronchial asthma includes inhibiting the release of myotonic mediators (particularly leukotrienes), calcium antagonism and preventing or reverting the beta-adrenergic receptor hyposensitivity (tachyphylaxis). Other properties of ketotifen that could contribute to its anti-asthmatic activity are: it blocks the migration of eosinophils to the inflammation site and suppresses their activation through the action of interleukins (cytokines); inhibits the development of airway hyperreactivity associated with the platelet-activating factor (PAF) or caused by vagal nerve activity after the exposure to an allergen in the presence of injury in the bronchial epithelium.

In the state of the art, US Patent 6,383,471 describes a pharmaceutical composition comprising: (a) a hydrophobic therapeutic agent with at least one ionizable functional group and an intrinsic solubility in water of at least less than 1% by weight, which can be selected from montelukast, ketotifen and pharmaceutically acceptable salts thereof; and (b) a vehicle comprising: (i) a ionizing agent capable of ionizing at least one ionizable functional group, in which the ionizing agent is present in a quantity of at least 0.1 molar equivalent per mole of at least one functional group; (ii) a surfactant selected from a group consisting of non-ionic hydrophilic surfactants having an HLB value equal or greater approximately 10, ionic hydrophilic surfactants, hydrophobic surfactants having an HLB value of less than 10, and mixtures thereof; and (iii) a triglyceride; and a method to prepare said composition, in which the dosage form is selected from the group consisting of a capsule, a solution, a cream, a lotion, an ointment, a suppository, a spray, an aerosol, a paste and a gel; and it is administered by route selected from the group consisting of oral, parenteral, topical, transdermal, ocular, pulmonary, vaginal, rectal and transmucosal; US Patent 8,377,994 describes a pharmaceutical composition and the use of roller-compacted pyrogenic produced silicon dioxide in pharmaceutical compositions; and US Patent 7,815,936 describes a pharmaceutical composition comprising granular pyrogenically produced silicon dioxide, having void volumes, and at least one pharmaceutical active constituent selected from montelukast, ketotifen or their derivatives, wherein the granular silicon dioxide has meso- and macropores, a mean grain diameter of 10 to 120 µm and a BET surface of 40 to 400 m²/g, determination according to DIN 66 131 using nitrogen and the mesopores account for 10 to 80% of the total volume and the particle size distribution of the granular material is 80 vol. % greater than 8 µm and 80% less than 96 µm and the proportion of the pores less than 5 µm be at most 5% referred to the total pore volume, and further comprises at least one pharmaceutical auxiliary substance is selected from: antioxidants, binders, emulsifiers, coloring agents, film-forming agents, fillers, gel-forming agents, odoriferous substances, flavoring substances, preservatives, solvents, oils, powder bases, ointment bases, acids and salts for the formulation, replenishment and production of pharmaceutical compositions, lubricants, release agents, suppository bases, suspension stabilizers, sweetening agents, effervescent gases, emollients or sugar substitutes; US Patent 8,268,352 describes modified release dosage form comprising of a high solubility active ingredient selected from montelukast sodium, prepared by using dual retard technique to control the release of the high solubility active ingredient, wherein the said dual retard technique is a combination of matrix formulation and reservoir formulation said dosage form consists of a) micro matrix particles which contain a high solubility active ingredient and one or more hydrophobic release controlling agents, and b) a coating of one or more hydrophobic release controlling agents on said micro matrix particles to form a double barrier to control the diffusion of the high solubility active ingredient, wherein the hydrophobic release controlling agents are selected from the group consisting of poly(ethyl acrylate, methyl methacrylate, trimethylammonioethyl methacrylate chloride) 1:2:0.1; poly(ethyl acrylate, methyl methacrylate, trimethylamonioethyl methacrylate chloride) 1:2:0.2 and poly(ethyl acrylate, methyl methacrylate) 1:1, polyvinyl acetate dispersion, ethylcellulose, cellulose acetate, lower molecular weight cellulose propionate, medium molecular weight cellulose propionate, higher molecular weight cellulose proprionate, cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly (hexyl methacrylate), poly(isodecyl methacrylate), poly (lauryl methacrylate), poly(phenyl methacrylate), poly (methyl acrylate), poly (isopropyl acrylate), poly (isobutyl acrylate), poly (octadecyl acrylate), beeswax, carnauba wax, microcrystalline wax, and ozokerite; cetostearyl alcohol, stearyl alcohol; cetyl alcohol and myristyl alcohol; glyceryl monostearate; glycerol monooleate, acetylated monoglycerides, tristearin, tripalmitin, cetyl esters wax, glyceryl palmitostearate, glyceryl behenate and hydrogenated castor oil, wherein the active ingredient can be less than or equal to 1500 mg, wherein said composition can additionally comprise another active ingredient such as ketotifen fumarate or its acceptable pharmaceutical salts as an immediate release form or a modified release form, said invention relates to the use of metformin hydrochloride in the same double retard technique to control its release; US 8,715,730 describes a stable orally disintegrating coated tablet containing at least one drug selected from montelukast, ketotifen, and pharmaceutically acceptable salts and solvates thereof, said tablet is coated with a coating layer containing a water-soluble substance and a polyvinyl alcohol resin in an amount of 10 to 60% by weight based on the weight of said coating layer, wherein the weight ratio of said polyvinyl alcohol resin in said coating layer to said water-soluble substance is 1:0.2 to 1:6, and wherein said water-soluble substance is at least one of maltose, maltitol, sorbitol, xylitol, fructose, glucose, lactitol, isomaltose, lactose, erythritol, mannitol, trehalose, sucrose, and polyethylene glycol having an average molecular weight of not more than 400 and wherein said coating layer disintegrates in an oral cavity within 18 seconds and said orally disintegrating tablet disintegrates in an oral cavity within 60 seconds; US Patent 8,758,816 describes a composition of matter comprising: (a) a single unit dosage of a pharmaceutical composition comprising montelukast and ketotifen; and (b) a unit dosage container comprising: (i) a squeezable chamber holding said single unit dosage and having an opening; and (ii) a closure mechanism removably attached to said opening of said squeezable chamber, wherein said unit dosage container is made of high density polyethylene (HDPE), wherein said single unit dosage of said pharmaceutical composition has a volume of less than about 0.6 ml, wherein said squeezable chamber has a volume of less than or equal to about 1.0 ml, wherein when said closure mechanism is removed, said unit dosage is retained in said squeezable chamber when said unit dosage container is inverted, and wherein when said squeezable chamber is squeezed, said squeezable chamber is deformed; US Patent 9,730,921 describes a method of inhibiting vascular leakage in a subject having a Dengue virus infection, the method comprising: (a) obtaining a biological sample from the subject; (b) determining the level of chymase in the biological sample from the subject; (c) comparing the level of chymase in the biological sample to a reference level of chymase; (d) identifying the subject as having a Dengue virus infection if the level of chymase is greater than the reference level of chymase; and (e) administering a mast cell modulator to the subject, wherein the mast cell modulator comprises cromolyn, ketotifen, or montelukast.

The present invention is characterized in that it provides a synergistic combination not previously reported in the state of the art, comprising montelukast or its pharmaceutically acceptable salts and ketotifen or its pharmaceutically acceptable salts. The potential advantage of using such a combination therapy is that it induces a significant blocking of LTD4 and histamine-induced contraction, and also minimizes the incidence of adverse effects. This combination of drugs allows the patient to promptly return to his/her daily activities. The use of this drug combination offers a synergism, thus reducing the required doses and minimizing adverse effects.

### OBJECT OF THE INVENTION

To provide a novel therapeutic option for the control and treatment of atopic diseases such as asthma, allergic rhinitis and atopic dermatitis, capable of reducing symptomatology and improving the quality of life of patients. To provide the usefulness of the montelukast and ketotifen combination, administered with pharmaceutically acceptable excipients or adjuvants, formulated as a single dosing unit to be administered orally in a solid or liquid form for the pharmacologic management of asthma, wherein the combination generates a synergic interaction, thus increasing its therapeutic power, shortening the onset of action period and reducing adverse events.

The combination of these active ingredients results in greater pharmacological potency, improving therapeutics and offering benefits such as: the administration of the active ingredients at lower concentrations than when separately administered, greater effectiveness and therapeutic potency, and significantly reducing the probability of side effects that could occur if the active ingredients were independently administered compared to when administered jointly.

### BRIEF DESCRIPTION OF THE DRAWINGS

Figure 1. Dose-response curve of LTD4-induced bronchoconstriction.
Figure 2. Dose-response curve of histamine-induced bronchoconstriction.
Figure 3. Antagonistic effect of LTD4-induced bronchoconstriction response in the presence of different cumulative concentrations of montelukast (0.003 to 30 µM). The control response is a vehicle.
Figure 4. Antagonistic effect of histamine-induced bronchoconstriction response in the presence of different cumulative concentrations of ketotifen (0.1 to 100 µM). The control response is a vehicle.
Figure 5. Dose-response relation of Montelukast combined with ketotifen and the relaxation of the bronchoconstriction effect induced by a histamine and LTD4 challenge.
Figure 6. Isobologram analysis of the bronchodilation effect of the montelukast-ketotifen interaction with different pharmacokinetic actions. The result is an interaction index of 0.35.

### DETAILED DESCRIPTION

Presently the treatment of asthma is complex, since it involves a specific process including: regular clinical follow-up, education on self-control, measures to avoid triggering factors and of course pharmacologic treatment, which is insufficient, its efficacy is unpredictable, dosing can be cumbersome and adverse effects are common. In the present invention, preclinical tests have proved that particular doses of the novel montelukast and ketotifen combination exhibit an unexpected and vigorous synergic therapeutic effect in the treatment of atopic diseases such as asthma, allergic rhinitis and atopic dermatitis. For this reason, the main object of the present invention is developing a pharmaceutic composition comprising the combination of a leukotriene receptor antagonist. i.e. the active ingredient montelukast and pharmaceutically acceptable salts thereof, and a histamine H1 inverse agonist, i.e. the active ingredient ketotifen and a pharmaceutically acceptable salt thereof, administered with pharmaceutically acceptable additives and/or excipients and/or adjuvants, formulated in a single dosing unit to be orally administered as a solid or liquid, indicated for the treatment and control of atopic diseases such as asthma, allergic rhinitis and atopic dermatitis.

Presently, an alternative to increase the efficacy of an analgesic treatment and to significantly reduce the secondary effects is through the combined administration of two or more active agents, such as the synergic pharmaceutical combination intended to be protected by the present invention. The montelukast and ketotifen combination has been designed for controlling symptoms, preventing the risk of future exacerbations and achieving the optimal function of the lung as the main organ, with minimal adverse or secondary effects caused by the pharmacological treatment.

The present invention intends to offer a new therapeutic option for the treatment and control of atopic diseases such as asthma, allergic rhinitis and atopic dermatitis, capable of reducing the symptomatology and improving the patient quality of life. The combination of the aforementioned active principles results in a greater pharmacological power.

The treatment for asthma and acute respiratory attack must be individualized, stepped-up or -down and continually adjusted in order to allow the patient in need of treatment to obtain a better control of the disease while minimizing adverse effects. This invention is characterized by the fact that the administration of the combination can be stepped-up for a period of time along with clinical visits and self-control (an important responsibility of the patient) in order to avoid a poor evolution (an incorrect evolution can be a consequence of inadequate compliance with the treatment, triggering factors or the presence of other diseases).

Representatively, at least one leukotriene-receptor antagonist or at least one histamine H1 inverse agonist are given to the patient for this treatment. The leukotriene-receptor antagonist is selected from the group consisting of albuterol sulphate, aminophylline, amoxicillin, ampicillin, astemizole, attenuated tubercle bacilli, azithromycin, bacampicillin, beclomethasone dipropionate, budesonide, bupropion hydrochloride, cefaclor, cefadroxil, cefixime, cefprozil, cefuroxime axetil, cephalexin, ciprofloxacin hydrochloride, clarithromycin, clindamycin, cloxacillin, doxycycline, erithromycin, ethambutol, fenotherol hydrobromide, fluconazole, flunisolide, fluticasone propionate, formoterol fumarate, gatifloxacin, flu virus vaccine, ipratropium bromide, isoniazid, isoproterenol hydrochloride, itraconazole, ketoconazole, ketotifen, levofloxacin, minocycline, montelukast, moxifloxacin, nedocromil sodium, nicotine, nystatin, ofloxacin, orciprenaline, oseltamivir, oseltamivir sulphate, oxtrifilin, penicillin, pirbuterol acetate, pivampicillin, pneumococcal conjugate vaccine, pneumococcal polysaccharide vaccine, prednisone, pirazinamide, rifampin, salbutamol, salmeterol xinafoate, cromoglycate sodium, terbutaline sulphate, terfenadine, theophylline, triamcinolone acetonide, zafirlukast, and zanamivir, while the histamine H1 inverse agonist can be selected from the group consistintg of azelastine, acrivastine, cyclizine, carebastine, cyproheptadine, carbinoxamine, doxylamine, dimetindene, ebastine, epinastine, efletirizine, ketotifen, levocabastine, mizolastine, mequitazine, mianserine, noberastine, meclizine, norastemizole, olopatadine, picumast, tripelennamine, temelastine, trimeprazine, triprolidine, brompheniramine, chlorpheniramine, dexchlorpheniramine, triprolidine, clemastine, diphenhydramine, diphenylpiraline, tripelennamine, hydroxyzine, methdilazine, promethazine, trimeprazine, azatadine, cyproheptadine, antazoline, pheniramine, pyrilamine, astemizole, terfenadine, loratadine, cetirizine, levocetirizine, fexofenadine, descarboethoxyloratadine, desloratadine, dimenhydrinate, hydroxyzine, and pharmaceutically acceptable salts, esters and derivates thereof.

The pharmaceutical combination in its base form or its pharmaceutically acceptable salts can be administered by any adequate route, by inhalation or intravenous, although more preferably orally as a solid or liquid.

Presently the effects that could be caused by the montelukast and ketotifen combination in the treatment of asthma have not been determined. Therefore, the object of this invention is to assess the efficacy of these pharmaceutical agents' combination in an in vitro model of rat tracheal rings constriction.

This combination improves the therapeutics and offers benefits such as: the administration of the active principles in lesser concentrations than when administered separately; more effectiveness, greater therapeutic power and significantly less probabilities of secondary effects when administered in a combination than when administered separately. Moreover, there is a reduction of secondary effects caused by the separate administration of the compounds, since the doses are lower than those commercially available. Accordingly, the behavior of this combination was proved preclinically, and the interaction and synergism of both compounds were determined along with the optimal proportions of the combination, which had a high degree of efficacy and therapeutic potentiation.

### Experimental model

### Experiment animals and operational description

Male Wistar rats 300-350 g were sacrificed with a phenobarbital sodium overdose given by intraperitoneal route. The thoracic cavity was exposed, the neck was dissected, and the trachea was removed 1 mm above the tracheal bifurcation and 1 mm below the thyroid cartilage. The sample was submerged in cold Krebs-Henseleit solution bubbled with 95% O2 and 5% CO2, and 3 mm-thick rings were cut. Each ring was placed in an isolated organ chamber, with its bottom anchored by means of a stainless-steel hook and another hook connected to a tension transducer. The chamber was then filled with Krebs-Henseleit solution at 36°C bubbled with 95% O2 and 5% CO2, pH 7.4 ± 0.5. Two washes were performed, and a 1.5 g load was applied to each ring and were left to rest for 60 minutes. The solution was replaced every 15 minutes and the rings were adjusted until a 1.5 g load was maintained on each ring.

Once concluded the rest period, the constricting agonists were administered in a cumulative concentration, with a 5 minutes stabilization period for each assay. Once the maximum concentration was achieved, the preparation was washed by replacing three times the solution in the chamber, and the preparation was left to rest for another 15 minutes. Acetylcholine was added to each ring at a concentration of 0.1 mM in the chamber in order to normalize the constricting effect induced by the agonists; the acetylcholine effect accounted for a 100 percent of the tracheal ring constriction.

### Agonist compounds used in the experiments

The following compounds were employed:

**Table 1. Leukotriene D4 final concentrations in the chamber**

| | |
|---|---|
| 0.1 nM | 0.0496 ng/mL |
| 1 nM | 0.4967 ng/mL |
| 10 nM | 4.967 ng/mL |
| 100 nM | 49.670 ng/mL |
| 1 µM | 0.4967 µM |
| 10 µM | 4.967 µM |

**Table 2. Histamine final concentrations in the chamber**

| | |
|---|---|
| 1 nM | 0.1112 ng/mL |
| 10 nM | 1.112 ng/mL |
| 100 nM | 11.115 ng/mL |
| 1 µM | 111.15 ng/mL |
| 5 µM | 555.75 ng/mL |
| 10 µM | 1.112 µg/mL |
| 100 µM | 11.115 µg/mL |

The antagonist's effects to their own agonists were analyzed using dose-response curves of the agonists in tracheal rings incubated with different antagonists' concentrations. The agonists were added at the end of the resting period, and 5 minutes later the agonists were added in cumulative concentrations. Once reached the maximum concentration, the preparation was washed replacing three times the solution in the chamber, and 15 minutes later acetylcholine was added to each ring at a 0.1 mM concentration in the chamber.

The analyzed concentrations of the antagonists were as follows:

**Montelukast final concentrations in the chamber**

| | |
|---|---|
| 0.003 µM | 1.759 ng/mL |
| 0.03 µM | 17.59 ng/mL |
| 0.3 µM | 175.9 µg/mL |
| 3.0 µM | 1. 759 µg/mL |
| 30.0 µM | 17.59 µg/mL |

**Ketotifen final concentrations in the chamber**

| | |
|---|---|
| 0.01 µM | 3.094 ng/mL |
| 0.1 µM | 30.94 ng/mL |
| 1.0 µM | 309.43 µg/mL |
| 10.0 µM | 3.094 µg/mL |
| 100.0 µM | 30.94 µg/mL |

The obtained data were graphed as a percentage of the final concentration compared with that of acetylcholine 0.1 mM, which was 100%.

The half maximal effective concentration (EC₅₀) of each dose-response curve of the analyzed agonists was obtained using the sigmoid model, wherein: y = a+ (b-a) / (1 +10 ^{((logx0-x)*p)}). The antagonists effect percentage curve was constructed with the displacement obtained in the concentration-response curves of an agonist in the presence of an antagonist.

Using 40 percent of the contraction inhibition effect induced by each agonist, the theoretical and experimental concentrations to be assessed for the 1:1 montelukast and ketotifen combination were calculated. These concentrations were evaluated in isolated rat tracheal rings contracted with the leukotriene D4 and histamine combination at a 1:1 proportion inducing 40 percent of the contraction.

Data are presented as mean ± standard deviation of 2 rings obtained from each of the tracheas of 5 rats. Data were analyzed with the one-way ANOVA test with a Dunett's post-test. EC50's were determined by means of the sigmoid model and the 40 percent of effects was obtained using a linear regression. The t and T values were determined based on a t-values table.

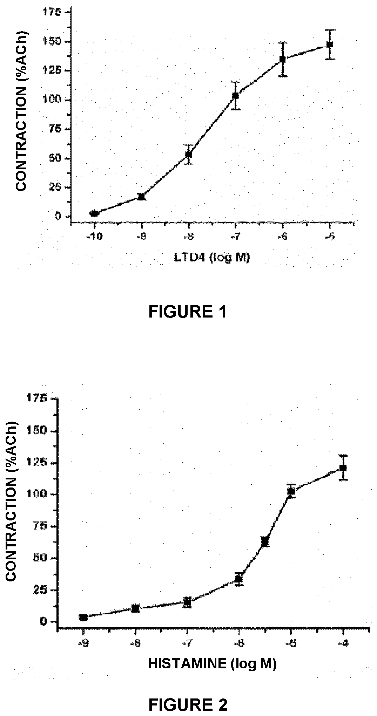

As a result, the cumulative leukotriene D4 (LTD4) administration induces a concentration-dependent contraction of the rat tracheal rings, this effect has a sigmoid behavior, and the highest concentrations caused a greater contraction than the one induced by acetylcholine 0.1 mM. In this experimental model, the LTD4 EC50 is 42.6 nM. (Figure 1).

The cumulative histamine administration induces a concentration-dependent contraction of the rat tracheal rings, this effect has a sigmoid behavior, and this effect has a sigmoid behavior. In this experimental model, the histamine EC50 is 3.54 µM. (Figure 2).

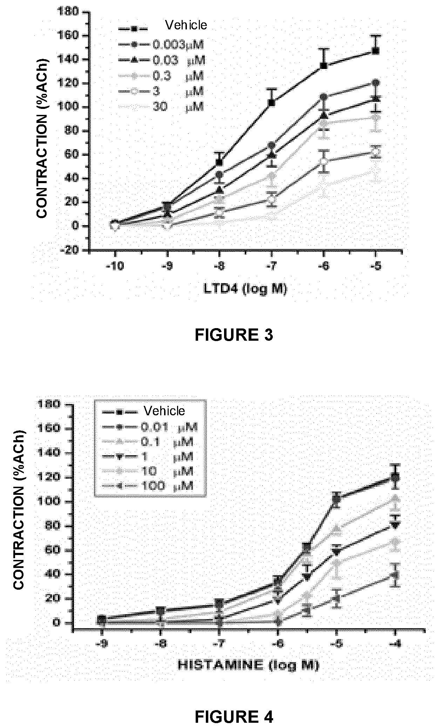

The presence of montelukast, a cysLT1 receptors antagonist, induces a concentration-dependent blocking of the contraction induced by the cumulative administration of LTD4. The assayed 30 µM montelukast concentration blocks 75% of the contraction induced by the highest LTD4 concentration. (Figure 3).

The presence of ketotifen, a H1 receptors antagonist, induces a concentration-dependent blocking of the contraction induced by the cumulative administration of histamine. The assayed 100 µM ketotifen concentration blocks 65% of the contraction induced by the highest histamine concentration. (Figure 4).

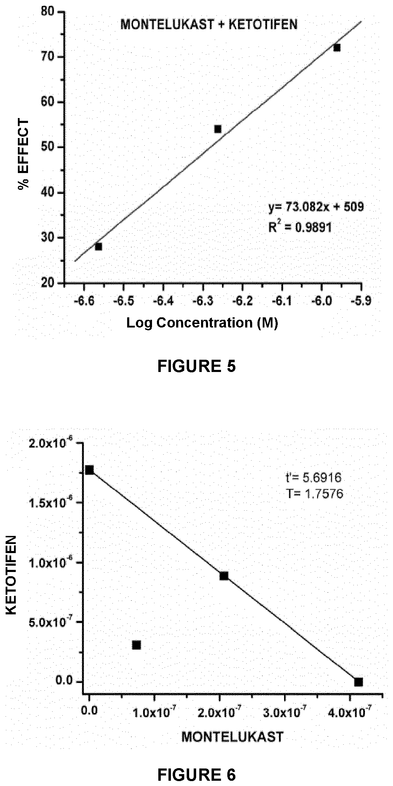

Figure 5 depicts the blocking percentage of the contraction induced by the combined administration of LTD4 and histamine (1:1) causing 40% of the maximum contraction, in the presence of the experimental concentrations of the montelukast and ketotifen combination (1:1).

Based on these results, an isobologram analysis was performed of the 1:1 montelukast- ketotifen combination blocking the contraction induced by LTD4 and histamine on rat tracheal rings. The obtained experimental data lie under the experimental calculation line, indicating a statistically significant synergic effect of the antagonist combination. (Figure 6).

According to this analysis, we can substantiate the fact that the administration of the 1:1 montelukast and ketotifen combination significantly blocks the contraction induced by LTD4 and histamine on rat tracheal rings. Hence, we can determine that this combination could be assessed in models of allergen-induced asthma, and to consider clinical studies of its potential introduction to the treatment of asthma in human beings.

The synergic interaction of the montelukast-ketotifen combination in a model of bronchoconstriction induced by a histamine-LTD4 challenge offers several possibilities: its potential use in the treatment of patients with bronchial asthma as a prophylaxis of relapses in the control of asthma. Also, it offers the possibility of reducing the doses or requirements of the active agents and nevertheless achieving the same therapeutic effect, thus reducing the frequency and/or severity of adverse events. This requires corresponding evidences derived from controlled clinical trials of the therapeutic efficacy in asthmatic patients.

In the current state of the art there are several pharmacological treatments of asthma and other derived diseases; however, there is no other treatment characterized by the combination of the active agents montelukast and ketotifen. The development of this invention provides a real and safe alternative for the control and treatment of asthma, and can reduce treatment durations, therapeutic effects and secondary reactions. The separate administration of these compounds requires approximately 0.01 mg to approximately 1000 mg/day for montelukast and approximately 0.01 mg to approximately 1000 mg/day for ketotifen.

This invention was developed to be administered by oral, nasal, intramuscular and/or intravenous route, either as immediate release of both drugs, or modified release of one or both drugs, with lower doses, higher therapeutic power and less risk of adverse events.

### EXAMPLES

The following pharmaceutic compositions are described below as nonlimiting examples:

### Example 1: Oral administration composition.

| |
|---|
| Montelukast or pharmaceutically acceptable salt |
| Ketotifen or pharmaceutically acceptable salt |
| Pharmaceutically acceptable excipient and/or carrier and/or additive |

### Example 2: Intramuscular and intravenous administration composition.

| |
|---|
| Montelukast or pharmaceutically acceptable salt |
| Ketotifen or pharmaceutically acceptable salt |
| Pharmaceutically acceptable excipient and/or carrier and/or additive |

### Example 3: Nasal administration composition.

| |
|---|
| Montelukast or pharmaceutically acceptable salt |
| Ketotifen or pharmaceutically acceptable salt |
| Pharmaceutically acceptable excipient and/or carrier and/or additive |

This invention can be represented in other specific ways without departing from its spirit or essential characteristics, and with any required excipient and/or carrier and/or additive to obtain the desired pharmaceutical form.

The described embodiments will be in any aspect considered as illustrative and not as limitations. Accordingly, the scope of this invention is defined by the appended claims and not by the above description. Its scope encompasses all modifications within the meaning and range of equivalence of the claims.

In an integral way, this invention provides the following advantages:
1. The combined administration of montelukast and ketotifen in a 1:1 ratio induces a significant blocking of the contraction induced by LTD4 and histamine in rat tracheal rings.
2. The potential therapeutic use for patients with atopic diseases such as bronchial asthma, allergic rhinitis and atopic dermatitis, as a prophylaxis of relapses in the control of asthma.
3. Offers the possibility of reducing the doses or requirements of the active agents and nevertheless achieving the same therapeutic effect, thus reducing the frequency and/or severity of adverse events.

## Claims

1. A synergic pharmaceutical combination **characterised in that** it comprises:
i. a leukotriene-receptor antagonist agent and/or pharmaceutically acceptable salts thereof,
ii. a histamine H1 inverse agonist agent and/or pharmaceutically acceptable salts thereof,
iii. a pharmaceutically acceptable carrier and/or excipient and/or additive, formulated as a single dosing unit to be administered by oral, nasal, intravenous or intramuscular route, indicated for the treatment and control of an atopic disease such as asthma, allergic rhinitis and atopic dermatitis; wherein the leukotriene-receptor antagonist agent is preferably montelukast or pharmaceutically acceptable salts thereof, and the histamine H1 inverse agonist agent is preferably ketotifen or pharmaceutically acceptable salts thereof.

2. The combination of claim 1 wherein the active agent montelukast is in a concentration of approximately 0.01 to approximately 1000 mg.

3. The combination of claim 1 wherein the active agent ketotifen is in a concentration of approximately 0.01 to approximately 1000 mg.

4. The combination of claim 1, useful for the preparation of a drug for the treatment of an atopic disease such as asthma, allergic rhinitis and atopic dermatitis in mammals.

5. The combination of claim 1, useful for the preparation of a drug formulated as a single dosing unit to be administered orally as a capsule, tablet, lozenge, sublingual tablet, granules, caplet, suspension or solution; in injectable form, intramuscularly or intravenously; or in devices for oral or nasal inhalation.

6. The combination of claim 1 wherein the mammal is a human.

7. A method of treating an atopic disease such as asthma, allergic rhinitis and atopic dermatitis comprising administering to mammals suffering the disease an effective amount of the combination of at least one compound selected from the group of leukotriene-receptor antagonist agents, preferably montelukast or pharmaceutically acceptable salts thereof, and at least one compound selected from the group of histamine H1 inverse agonist agents, preferably ketotifen or pharmaceutically acceptable salts thereof.

8. A method of treating asthma according to claim 7 wherein the compounds are administered orally, nasally, intramuscularly and/or intravenously.

9. A method of treating asthma according to claim 8 wherein the administration of the compounds is in an amount of approximately 0.01 mg to approximately 1000 mg/day of montelukast or pharmaceutically acceptable salts thereof, and approximately 0.01 mg to approximately 1000 mg/day of ketotifen or pharmaceutically acceptable salts thereof.
